# EUROPEAN PATENT APPLICATION

(11) **EP 3 960 243 A1**
(43) Date of publication of application: **02.03.2022**
(21) Application number: 20796282.0
(22) Date of filing: 24.04.2020
(51) Int. Cl.: A61P 35/00, A61K 31/195, A61K 41/00

(54) **PHARMACEUTICAL COMPOSITION FOR PREVENTING OR TREATING TREATMENT-RESISTANT CANCER**

(30) Priority: 26.04.2019 WO PCT/JP2019/017995
(71) Applicant: Jimro Co., Ltd., Takasaki-shi, Gunma 370-0021 (JP)
(72) Inventor: KOHASHI, Masayuki., Hirakata-shi, Osaka 573-1132 (JP); HARADA, Yasuo, Osaka-shi, Osaka 540-0021 (JP)
(74) Representative: dompatent von Kreisler Selting Werner - Partnerschaft von Patent- und Rechtsanwälten mbB
(86) International application number: PCT/JP2020/017805
(87) International publication number: WO 2020/218562

(57) **Abstract**

Provided are a pharmaceutical composition for treating or preventing drug-resistant cancer by photodynamic therapy (PDT) that contains 5-aminolevulinic acid (ALA) or a derivative thereof, or a salt of these, and a method for treating or preventing drug-resistant cancer using the pharmaceutical composition.

## Description

### Technical Field

### Cross-Reference to Related Application

The present application claims the benefits of priority of international application No. PCT/JP2019/17995 filed to the Japan Patent Office as the receiving office on April 26, 2019, said application is incorporated herein by reference in their entirety.

The present invention relates to a pharmaceutical composition including 5-aminolevulinic acid, for treating or preventing drug-resistant cancer.

### Background Art

5-aminolevulinic acid (5-ALA) is an orally absorbed substance which is metabolized to protoporphyrin IX (PpIX) during the biosynthesis of heme in intracellular mitochondria. The PpIX is a photosensitive substance which generates reactive oxygen species when irradiated with light ray in certain absorption band, which is called as Soret band (410 nm) or Q band (500-650 nm). Therefore, cell death can be induced by irradiating a cell in which PpIX is accumulated with light having absorption band wavelength(s) (Non-Patent Document 1). A method of treatment based on the principle of this cell-killing effect is called as photodynamic therapy (PDT), and the PDT using 5-ALA is expected to be applied clinically in the future (Patent Documents 1 and 2).

According to the "2016 Summary of Vital Statistics" released by the Ministry of Health, Labor and Welfare in Japan, the death rate from malignant neoplasms in Japan is 28.5% of all deaths, which is the first in the list of causes of death. For an early-stage cancer, surgical total removal of tumor is primary curative, and the response rate is high. On the other hand, for an advanced cancer, a treatment with anticancer drug(s) is usually carried out.

However, it is known that anticancer drugs may be ineffective from the beginning of treatment, and that the effects of anticancer drugs are often transient even in the drug-response cancer and further the effects of anticancer drugs decrease during the course of treatment, and at the same time the cancer may acquire resistance to anticancer drugs with different mechanism of action and structures (Non-Patent Documents 2 and 3). These drug-resistant cancer cells (including cancer stem cells) may respond poorly to chemotherapy, and may cause metastasis and recurrence when the cells grow back.

The existence of drug-resistant cancer cell is still one of the major factors preventing an improvement of clinical outcomes in the overall treatment of cancer today. Although mechanisms of acquiring drug-resistance by cancer cell are complex and multiple, a cancer patient who does not show sufficient sensitivity to anticancer drug(s) is clearly classified in each clinical treatment algorithm and distinguished disease state from an anticancer drug-sensitive cancer, and the treatment policy to be taken will be different from those for an anticancer drug-sensitive cancer (Non-Patent Document 4).

The above-mentioned acquiring cross resistance by cancer cell to anticancer drug(s) with different mechanisms of action and structures same applies to PDT. In general, in PDT including usage of 5-ALA, there are many reports that PDT is often ineffective against a cancer which is resistant to anticancer drug(s), and then it is known that either anticancer drug resistance and PDT resistance are crossed and both acquired (Non-Patent Document 5). This means that PDT does not necessarily have a cell-killing effect in cancer cell which is resistant to an anticancer drug, and the efficacy of PDT against various anticancer drug-resistant cancers cannot be easily predicted.

In addition, as shown in Non-Patent Document 5, some photosensitizers are effective in killing cell against a cancer which is resistant to the same anticancer drug, while others are ineffective. Therefore, even if PDT using a particular photosensitizer is effective against drug-resistant cancer, it is not easy for a person skilled in the art to predict whether or not PDT using another photosensitizer will also be effective. This is natural not only for drug-resistant cancer, and for example, a report shows that PDT using hypericin or Photofrin showed antitumor effect, while PDT using TPPS4 or 5-ALA did not show the effect (Non-Patent Document 6).

Patent Document 3 discloses a preventive or therapeutic composition for a therapy-resistant cancer having therapy-resistant cancer cells, which is for use in photodynamic therapy, wherein the composition comprises 5-ALA or salt thereof. However, this document only discloses that inhibiting ABCG2 enhances the accumulation of intracellular PpIX and enhances the effect of 5-ALA/PDT on therapy-resistant cancer cells through the finding that tyrosine kinase inhibitors inhibit ABCG2, which is involved in the efflux of PpIX. This document neither discloses that 5-ALA/PDT is effective for tyrosine kinase inhibitor-resistant cancer nor suggests the efficacy of 5-ALA/PDT in cancer resistant to other drugs.

The above-mentioned Patent Document 3 discloses a preventive or therapeutic composition for a therapy-resistant cancer having therapy-resistant cancer cells, which is for use in photodynamic therapy, wherein the composition comprises 5-ALA or salt thereof. The therapy-resistant cancer in Patent Document 3 is "preferably malignant neoplasm, but the type of the therapy-resistant cancer is not limited. Examples of the therapy-resistant cancer include primary or metastatic, invasive or non-invasive carcinomas or sarcomas, such as brain tumor, spinal cord tumor, maxillary sinus carcinoma, pancreatic ductal adenocarcinoma, carcinoma of gingiva, tongue carcinoma, lip carcinoma, nasopharyngeal carcinoma, oropharynx carcinoma, hypopharyngeal carcinoma, laryngeal carcinoma, thyroid carcinoma, parathyroid carcinoma, lung carcinoma, pleural tumor, carcinomatous peritonitis, carcinomatous pleurisy, esophageal carcinoma, stomach carcinoma, colon carcinoma, bile duct carcinoma, gallbladder carcinoma, pancreatic carcinoma, liver carcinoma, kidney carcinoma, bladder carcinoma, prostate carcinoma, penile carcinoma, testicular tumor, adrenal carcinoma, cervical carcinoma, uterine carcinoma, vaginal carcinoma, vulvar carcinoma, ovary carcinoma, bone tumor, breast carcinoma, skin carcinoma, melanoma, basal cell carcinoma, lymphoma, Hodgkin's disease, plasmacytoma, osteosarcoma, chondrosarcoma, liposarcoma, rhabdomyosarcoma, and fibrosarcoma. The therapy-resistant cancer is particularly preferably brain tumor. In addition, the therapy-resistant cancer is preferably solid cancer". Thus, Patent Document 3 does not indicate the efficacy of 5-ALA in the therapy-resistant cancer other than solid cancer, such as leukemia and blood cancer disease. In addition, Patent Document 3 practically only discloses the application of PDT to a brain tumor cell and a cervical cancer cell.

Lenalidomide is an anti-hematopoietic malignancy agent used in the treatment of multiple myeloma and other malignancies. Mogamulizumab is an antineoplastic agent used in the treatment of T-cell lymphoma and other malignancies. Tretinoin (all-trans retinoic acid, ATRA) is a leukemia treatment agent used for acute promyelocytic leukemia and other conditions. Imatinib is an antineoplastic agent used in the treatment of chronic myelogenous leukemia and other malignancies.

All references cited herein are incorporated by reference in their entirety. To the extent the material incorporated by reference is inconsistent with the present specification, the present specification will supercede any such material.

### Prior Art Documents

### Patent Documents

Patent Document 1: JP 2005-132766 A
Patent Document 2: JP 2017-513952 A
Patent Document 3: WO 2015/125732 A

### Non-Patent Documents

Non-Patent Document 1: T Namikawa et al, World J Gastroenterol. 21(29): 8769-8775, 2015
Non-Patent Document 2: Ueda K et al, Proc Natl Acad Sci USA, 84: 3004-3008, 1987.
Non-Patent Document 3: Tada Y et al, Int J Cancer, 98: 630-635, 2002
Non-patent document 4: Guidelines for the treatment of hematopoietic tumors edited by the Japanese Society of Hematology
Non-Patent Document 5: Casas A et al., Curr Med Chem. 18(16): 2486-515, 2011
Non-Patent Document 6: Luksiene Z et al, Medicina (Kaunas). 2003; 39(7): 677-82.

### Summary of Invention

### Problems to Be Solved by Invention

An object of the present invention is to provide a pharmaceutical composition for treating or preventing drug-resistant cancer.

### Means for Solving Problems

The present inventors have intensively studied to solve the above-mentioned problems. As a result, they have found that PDT using 5-ALA may effectively treat or prevent drug-resistant cancer. The present invention has been completed based on the findings.

That is, the present invention relates to:
(1) a pharmaceutical composition for treating or preventing drug-resistant cancer with photodynamic therapy (PDT), including 5-aminolevulinic acid (5-ALA) as shown in the following formula (1): 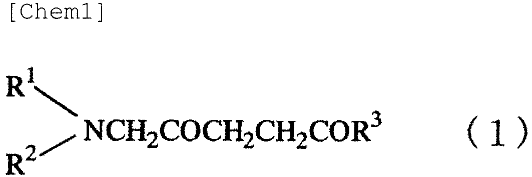 (where, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof, where the drug is an immunomodulator reducing an expression of interferon regulatory factor, or a molecular-targeting therapeutic agent targeting CD molecule, cereblon, mTOR protein, PML-RARα, BCR-Abl tyrosine kinase, or KIT tyrosine kinase;
(2) a pharmaceutical composition for treating or preventing drug-resistant leukemia with photodynamic therapy (PDT), including 5-aminolevulinic acid (5-ALA) as shown in the following formula (1): 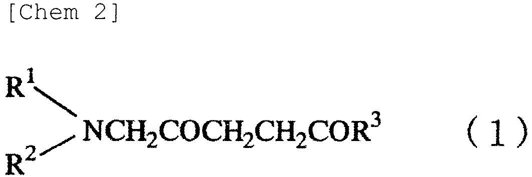 (where, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof;
(3) The pharmaceutical composition of (2), where the leukemia is adult T-cell leukemia;
(4) The pharmaceutical composition of any of (1) to (3), where the drug is lenalidomide or mogamulizumab;
(5) The pharmaceutical composition of any of (1) to (3), where the drug is tretinoin;
(6) The pharmaceutical composition of any of (1) to (3), where the drug is imatinib;
(7) a pharmaceutical composition for treating or preventing a cancer with photodynamic therapy (PDT), including 5-aminolevulinic acid (5-ALA) as shown in the following formula (1): 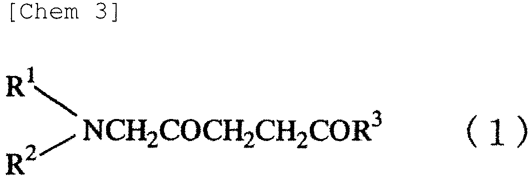 (where, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof, where the pharmaceutical composition is for use in combination with at least one of lenalidomide, mogamulizumab, tretinoin, and imatinib;
(8) The pharmaceutical composition of any of (1) to (7), where the cancer is a hematological cancer;
(9) The pharmaceutical composition of any of (1) to (8), where the PDT light irradiation is performed *in vitro.*

### Effect of Invention

By using the composition of the present invention, drug-resistant cancer may be treated or prevented.

### Brief Description of Drawings

[Fig. 1] Fig. 1 is graphs showing the cell viability of lenalidomide-resistant and mogamulizumab- and tretinoin-resistant cell lines.
[Fig. 2] Fig. 2 is graphs showing the cell viability of imatinib-resistant and doxorubicin-resistant cell lines.

### Mode for Carrying Out Invention

The present invention will be described in detail as follows. In one aspect, the present invention provides a pharmaceutical composition for treating or preventing drug-resistant cancer with photodynamic therapy (PDT), including 5-aminolevulinic acid (5-ALA) or derivative thereof, or salt thereof (also referred to herein as "5-ALAs").

As used herein, 5-ALA or derivative thereof is a compound having the following formula (1): 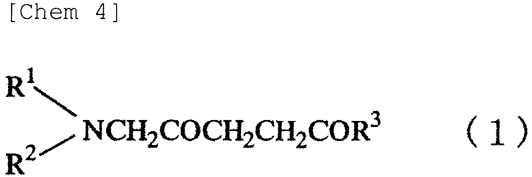 (In the formula, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group).

In the formula (1), examples of the alkyl group indicated by R¹ and R² include straight or branched chain alkyl group having 1 to 24 carbons, more specifically, alkyl group having 1 to 18 carbons and alkyl group having 1 to 6 carbons. More specific examples of the alkyl group include methyl, ethyl, n-propyl, isopropyl, n-butyl, and sec-butyl group. Examples of the acyl group include straight or branched chain alkanoyl or alkenyl carbonyl group, and aroyl group, having 1 to 12 carbons, and more specifically, alkanoyl group having 1 to 6 carbons. More specific examples of the acyl group include formyl group, acetyl group, propionic acid, and butyryl group. Examples of the alkoxycarbonyl group include alkoxycarbonyl group having total carbon number of 2 to 13, and more specifically, alkoxycarbonyl group having total carbon number of 2 to 7. More specific examples of the alkoxycarbonyl group include methoxycarbonyl group, ethoxycarbonyl group, n-propoxycarbonyl group, and isopropoxycarbonyl group. Examples of the aryl group include aryl group having 6 to 16 carbons, such as phenyl and naphthyl group. Examples of the aralkyl group include group in which aryl group having 6 to 16 carbons is bonded to alkyl group having 1 to 6 carbons, such as phenyl-C₁₋₆ alkyl group and naphthyl-C₁₋₆ alkyl group.

In one embodiment, R¹ and R² in the formula (1) are hydrogen atoms. In another embodiment, R³ is hydroxy group, alkoxy group, or aralkyloxy group, more specifically hydroxy group or C₁₋₁₂ alkoxy group.

The salt of 5-ALA or derivative thereof, is not particularly limited as long as the salt is pharmaceutically acceptable. Specific examples include acid addition salt of organic or inorganic acid, and more specifically, hydrochloride, hydrobromide, sulfate, phosphate, acetate, lactate, citrate, tartrate, succinate, maleate, fumarate, and ascorbate.

The 5-ALA or derivative thereof, or salt thereof, may be produced by the methods described in, for example, JPH04-9360 A and JPH11-501914 A.

The pharmaceutical composition of this aspect is used for a treatment or prevention of drug-resistant cancer. As used herein, drug resistance refers to that a treatment or prevention effect of the drug is not obtained or hard to be obtained. A drug-resistant cell may include both acquired and congenitally drug-resistant cells. In some embodiments, the drug is an immunomodulator, a molecular-targeting therapeutic agent, or an anticancer antibiotic. Examples of the immunomodulator include an agent which reduces an expression of interferon regulatory factor, more specifically, interferon regulatory factor 4. Examples of the molecular-targeting therapeutic agent include CD molecule such as CD20, CD22, CD33, CD52, and CD194 (CCR4), cereblon, mTOR protein, PML-RARα, BCR-Abl tyrosine kinase, and KIT tyrosine kinase. More specific examples include lenalidomide, mogamulizumab, rituximab, ofatumumab, alemtuzumab, gemtuzumab ozogamicin, brentuximab vedotin, ibritumomab tiuxetan, tretinoin, dasatinib, bosutinib, nilotinib, and imatinib. Examples of the anticancer antibiotic include anthracycline antibiotic, more specifically, doxorubicin. More specific examples of the drug include lenalidomide, mogamulizumab, tretinoin, and imatinib. Further specific examples include lenalidomide, mogamulizumab, and tretinoin.

The type of drug-resistant cancer is not particularly limited, and examples thereof include cancer, sarcoma, adenocarcinoma, lymphoma, leukemia, solid cancer, and lymphoma, of mammalian including or except human, or human. More specific examples of the cancer include lung cancer such as non-small cell lung cancer and NSCLC, ovarian cancer, prostate cancer, colorectal cancer, liver cancer, kidney cancer, bladder cancer, breast cancer, thyroid cancer, pleural cancer, pancreatic cancer, uterine cancer, cervical cancer, testis cancer, anal cancer, pancreatic cancer, bile duct cancer, gastrointestinal carcinoid tumor, esophageal cancer, gallbladder cancer, appendix cancer, small intestinal cancer, gastric cancer, central nervous system cancer, skin cancer, choriocarcinoma, head and neck cancer, hematological cancer, osteogenic sarcoma, fibrosarcoma, neuroblastoma, glioma, melanoma, B-cell lymphoma, non-Hodgkin's lymphoma, Burkitt's lymphoma, small cell lymphoma, large cell lymphoma, monocytic leukemia, myeloid leukemia, acute lymphocytic leukemia, acute myeloid leukemia, and multiple myeloma. More specific examples of the cancer include hematological cancer such as leukemia, lymphoma, multiple myeloma, mycosis fungoides, and Sezary's syndrome. Preferred examples include chronic myeloid leukemia and adult T-cell leukemia, especially adult T-cell leukemia.

The means of administration of the pharmaceutical composition of this aspect is not particularly limited, and includes, for example, oral administration, intravenous administration, intramuscular administration, local administration to the affected area, transdermal administration, and transrectal administration.

The dosage form of the pharmaceutical composition of this aspect is not particularly limited, and includes, for example, oral dosage form such as granule, fine granule, and tablet; injectable dosage form such as liquid and time-soluble powder; transdermal dosage form such as ointment, liquid, cream, and gel; and suppository.

In addition to 5-ALA or derivative thereof, or salt thereof, the pharmaceutical composition of this aspect may contain another pharmaceutically acceptable raw material, such as pharmaceutically acceptable carrier, excipient, diluent, isotonic agent, additive, disintegrant, binder, stabilizer, coating agent, dispersant, bulking agent, pH buffer, lubricant, flavoring agent, sweetener, solubilizer, solvent, gelling agent, and nutritional agent. The other raw material may affect the absorption and blood concentration of the pharmaceutical composition of this aspect, resulting in changes in pharmacokinetics. Specific examples of the other raw material include water, saline, animal fat, vegetable fat, lactose, starch, gelatin, crystalline cellulose, gum, talc, magnesium stearate, hydroxypropyl cellulose, polyalkylene glycol, polyvinyl alcohol, and glycerin.

The dosage of the pharmaceutical composition of this aspect may be determined by a person skilled in the art as long as an amount of PpIX accumulated in the targeted drug-resistant cancer cell is an effective amount in PDT. The dosage, timing, frequency, and duration of administration to a subject may be determined by a person skilled in the art depending on age, weight, symptom, and condition of the subject, as well as the condition and ease of administration of the cell, tissue, or organ in the subject to be prevented or treated. Examples of more specific dosage include about 1 mg to about 1,000 mg, about 5 mg to about 100 mg, about 10 mg to about 30 mg, about 15 mg to about 25 mg, per kilogram of body weight in terms of ALA.

The frequency of administration of the pharmaceutical composition of this aspect is not particularly limited, and examples include once or multiple times a day, such as two, three, four, or five times a day, and continuous administration by intravenous infusion. The period of administration of the pharmaceutical composition of this aspect may be determined by a person skilled in the art based on various clinical parameters, such as symptom or condition of the subject.

The pharmaceutical composition of this aspect is used in photodynamic therapy (PDT). PDT is a therapy that treats target area by administering a light-reactive compound and irradiating it with light ray. A typical example is PDT using 5-ALAs.

PDT after administration of the pharmaceutical composition of this aspect is carried out by irradiating light of specific wavelength(s). The wavelength(s) to be used may be determined by a person skilled in the art. Examples of wavelength used include about 350 nm to about 700 nm, about 400 nm to about 640 nm, about 480 nm to about 640 nm, 505 ± 10 nm, 540 ± 10 nm, 580 ± 10 nm, and 630 ± 10 nm.

The time from administration of the pharmaceutical composition of this aspect to start of irradiation is not particularly limited and may be set by a person skilled in the art according to the type of cancer, means of administration, etc. For example, light irradiation may be started from about 15 minutes to about 48 hours after the administration, and from about 1 to about 24 hours after the administration.

The number of irradiations and the irradiation time per irradiation in PDT are not particularly limited and may be determined by a person skilled in the art. For example, one or more irradiations, e.g., 2, 3, 4, or 5 to about 100 irradiations, may be performed. The light may be irradiated with the same wavelength or with different wavelengths. When the pharmaceutical composition of this aspect is administered multiple times, PDT may be performed with one or more irradiations between each administration.

The light intensity of the light to be irradiated in PDT may be set by a person skilled in the art according to the type of cancer, means of administration, light source, etc. Examples of the light intensity include from about 0.01 to about 200 J/cm², from about 5 to about 150 J/cm², from about 10 to about 100 J/cm², from about 30 to about 100 J/cm², from about 10 to about 30 J/cm², 10 ± 10 J/cm², 30 ± 10 J/cm², and 100 ± 10 J/cm².

When the target cancer is hematological cancer, the light irradiation in PDT may be performed outside the body. For example, after administering the pharmaceutical composition of this aspect, blood may be circulated extracorporeally by a blood perfusion device, and the blood in the perfusion device may be irradiated with light.

In some embodiments, the pharmaceutical composition of this aspect may be used in combination with another drug, such as anticancer drug. The other drug may be included in the pharmaceutical composition of this aspect, or as a combination of the pharmaceutical composition of this aspect and the drug. The pharmaceutical composition of this aspect may be administered and PDT may be performed after the drug is administered, or the drug may be administered after the pharmaceutical composition is administered and PDT is performed. The drug may also be administered between the administration of the pharmaceutical composition of this aspect and PDT, or between multiple PDTs after administering the pharmaceutical composition of this aspect. Examples of the other drug include anticancer agent such as tyrosine kinase inhibitor, immunomodulator, and molecular-targeting therapeutic agent. In addition, PDT with using the pharmaceutical composition of this aspect may be performed in combination with lenalidomide, mogamulizumab, tretinoin, imatinib, or doxorubicin, for example, in a subject who will be, was, or continues to be treated with lenalidomide, mogamulizumab, tretinoin, imatinib, or doxorubicin. In more specific embodiment, the drug used in combination is lenalidomide, mogamulizumab, tretinoin, or imatinib. In further specific embodiment, the drug used in combination is lenalidomide, mogamulizumab, or tretinoin. In some embodiments, the other drug does not include tyrosine kinase inhibitor. In other embodiments, the other drug does not include ABCG2 inhibitor.

In another aspect, the present invention relates to a kit for treating or preventing drug-resistant cancer with PDT, containing 5-ALAs. In another aspect, the present invention relates to a method of treating or preventing drug-resistant cancer with PDT using 5-ALAs. In another aspect, the present invention relates to 5-ALAs for use in treatment or prevention of drug-resistant cancer with PDT. In a further aspect, the present invention relates to a use of 5-ALAs for manufacturing a pharmaceutical composition for treatment or prevention of drug-resistant cancer with PDT. More specific embodiments of these aspects are as described in the aspect of the pharmaceutical composition.

As used herein, the description of a numerical range, e.g. "1 to 5 times" is understood as to represent any number within the range, such as 1, 2, 3, 4, and 5 each. As used herein, the term "about" refers to a range of ±10%, preferably ±5%. The values that serve as the boundaries of the range are considered to be described herein.

The present invention will be described in detail by way of examples. However, the present invention is not necessarily limited by the examples.

### Examples

### Example 1: Confirmation of cell killing effect in adult T-cell leukemia (ATL) cell line

ATN-1, C8166, TL-Om1, Hut102, and MT-1 were used as ATL cell lines. Among them, lenalidomide-sensitive cell line was Hut102 and lenalidomide-resistant cell line was C8166. The sensitivity of ATN-1, TL-Om1, and MT-1 to lenalidomide was unknown. Meanwhile, tretinoin-sensitive cell line was C8166 and tretinoin-resistant cell line was Hut102. The sensitivity of ATN-1, TL-Om1, and MT-1 to tretinoin was unknown. In addition, mogamulizumab-sensitive cell lines were ATN-1, TL-Om1, and MT-1, and mogamulizumab-resistant cell line was Hut102. The sensitivity of C8166 to mogamulizumab was unknown. These cells were passaged and cultured. For the experiment, cells were prepared to a density of 1 × 10⁶ cells/ml at the beginning of the experiment.

To the cell suspension, 5-ALA was added at concentrations of 0, 0.0625, 0.125, 0.25, and 0.5 mM and loaded for 4 hours. The cell after 5-ALA loading was washed and irradiated with light (0, 10, 30, and 100 J/cm²) at a central wavelength of 630 nm, and cell survival rate (%) was calculated by Cell Counting Kit-8.

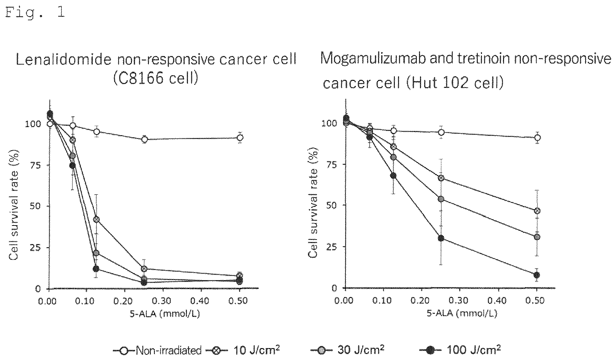

The result is shown in Fig. 1. As shown in Fig. 1, PDT using 5-ALA killed C8166 cell line, which was lenalidomide-resistant cell line, and HuT102 cell line, which was mogamulizumab-resistant cell line and tretinoin-resistant cell line. Thus, the result showed that PDT using 5-ALA was effective against these drug-resistant cancer cells.

With respect to the resistance to lenalidomide, each EC₅₀ value of 5-ALA and its 95% confidence interval to cell survival rate (%) at each irradiation energy density in C8166, which was resistant cell line, and Hut102, which was lenalidomide-sensitive cell line, were shown in Table 1.

**[Table 1]**

| | EC₅₀ (mmol/L) (95% confidence interval) | |
|---|---|---|
| | Lenalidomide-resistant cell line: C8166 | Lenalidomide-sensitive cell line: Hut102 |
| 10 J/cm² | 0.113 (0.100-0.128) | 0.441 (0.329-0.612) |
| 30 J/cm² | 0.087 (0.078-0.097) | 0.277 (0.200-0.396) |
| 100 J/cm² | 0.077 (0.069-0.086) | 0.173 (0.144-0.208) |

The results of each EC₅₀ value showed that the cell killing effect of PDT using 5-ALA in the lenalidomide-resistant cell line C8166 was comparable to that of the lenalidomide-sensitive cell line Hut102. Therefore, it was suggested that PDT using 5-ALA has an excellent cytotoxic effect even in lenalidomide-non-sensitive patient's cells, and at the same time, the combined use of lenalidomide and PDT using 5-ALA may also be effective.

With respect to the resistance to tretinoin, each EC₅₀ value of 5-ALA and its 95% confidence interval to cell survival rate (%) at each irradiation energy density in Hut102, which was resistant cell line, and C8166, which was tretinoin-sensitive cell line, were shown in Table 2.

**[Table 2]**

| | EC₅₀ (mmol/L) (95% confidence interval) | |
|---|---|---|
| | Tretinoin-resistant cell line: Hut102 | Tretinoin-sensitive cell line: C8166 |
| 10 J/cm² | 0.441 (0.329-0.612) | 0.113 (0.100-0.128) |
| 30 J/cm² | 0.277 (0.200-0.396) | 0.087 (0.078-0.097) |
| 100 J/cm² | 0.173 (0.144-0.208) | 0.077 (0.069-0.086) |

The results of each EC₅₀ value showed that the cell killing effect of PDT using 5-ALA in the tretinoin-resistant cell line C8166 was comparable to that of the tretinoin-sensitive cell line Hut102. Therefore, it was suggested that PDT using 5-ALA has an excellent cytotoxic effect even in tretinoin-non-sensitive patient's cells, and at the same time, the combined use of tretinoin and PDT using 5-ALA may also be effective.

With respect to the resistance to mogamulizumab, each EC 50 value of 5-ALA and its 95% confidence interval to cell survival rate (%) at each irradiation energy density in Hut102, which was resistant cell line, and ATN-1, TL-Om1, and MT-1, which were mogamulizumab-sensitive cell lines, were shown in Table 3.

**[Table 3]**

| | EC₅₀ (mmol/L) (95% confidence interval) | | | |
|---|---|---|---|---|
| | Mogamulizumab-sensitive cell line: ATN-1 | Mogamulizumab-sensitive cell line: TL-Om1 | Mogamulizumab-resistant cell line: Hut102 | Mogamulizumab-sensitive cell line: MT-1 |
| 10 J/cm² | 0.113 (0.105-0.121) | 0.269 (0.236-0.306) | 0.441 (0.329-0.612) | >0.5 |
| 30 J/cm² | 0.087 (0.083-0.093) | 0.141 (0.128-0.155) | 0.277 (0.200-0.396) | 0.202 (0.184-0.221) |
| 100 J/cm² | 0.084 (0.081-0.088) | 0.092 (0.085-0.100) | 0.173 (0.144-0.208) | 0.115 (0.107-0.123) |

The results of each EC₅₀ value showed that the cell killing effect of PDT using 5-ALA in the mogamulizumab-resistant cell line Hut102 was at least comparable to that of the other sensitive cell lines. Therefore, it was suggested that PDT using 5-ALA has an excellent cytotoxic effect even in mogamulizumab-non-sensitive patient's cells, and at the same time, the combined use of mogamulizumab and PDT using 5-ALA may also be effective.

### Example 2: Confirmation of cell killing effect in Chronic Myeloid Leukemia (CML) cell line

LAMA84-r was used as imatinib-resistant CML cell line and the original cell line LAMA84-s (imatinib-sensitive) was used as a comparative control. In addition, K562/ADR was used as doxorubicin-resistant CML cell line and the original cell line K562 (doxorubicin-sensitive) was used as a comparative control. These cells were passaged and cultured. For the experiment, cells were prepared to a density of 1 × 10⁶ cells/ml at the beginning of the experiment.

To the cell suspension, 5-ALA was added at concentrations of 0, 0.0625, 0.125, 0.25, and 0.5 mM and loaded for 4 hours. The cell after 5-ALA loading was washed and irradiated with light (0, 10, 30, and 100 J/cm²) at a central wavelength of 630 nm, and cell survival rate (%) was calculated by Cell Counting Kit-8.

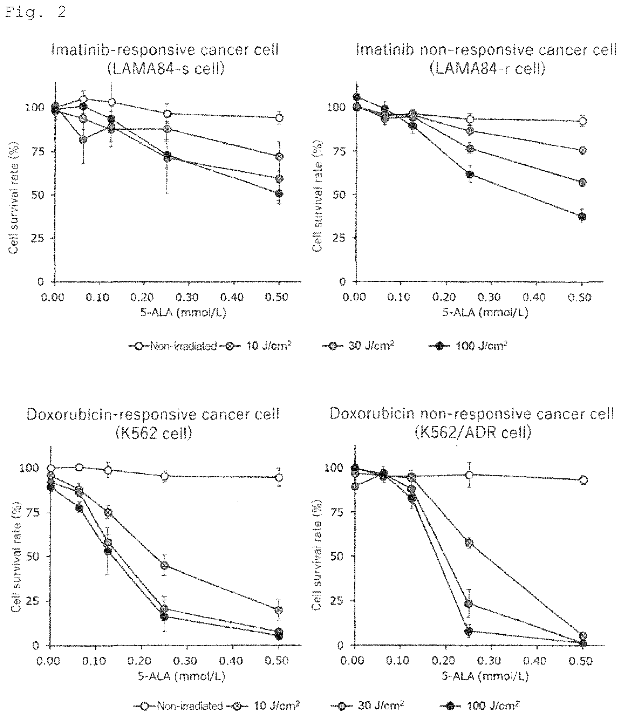

The results are shown in Fig. 2. As shown in Fig. 2, PDT using 5-ALA killed LAMA84-r cell line, which was imatinib-resistant cell line, and K562/ADR cell line, which was doxorubicin-resistant cell line. These cell killing effects were comparable to those of the respective sensitive cell lines. Therefore, it was suggested that PDT using 5-ALA was effective in imatinib- and doxorubicin-non-sensitive patient's cells.

### Industrial applicability

The present invention enables to prevent or treat drug-resistant cancer with PDT using 5-ALA. The present invention may be used in the medical field and other fields.

## Claims

1. A pharmaceutical composition for treating or preventing drug-resistant cancer with photodynamic therapy (PDT), comprising 5-aminolevulinic acid (5-ALA) as shown in the following formula (1): 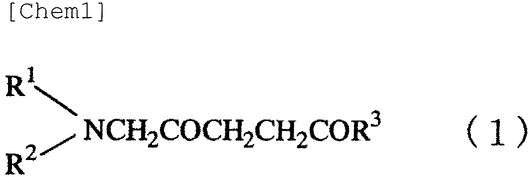 (wherein, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof, wherein the drug is an immunomodulator reducing an expression of interferon regulatory factor, or a molecular-targeting therapeutic agent targeting CD molecule, cereblon, mTOR protein, PML-RARα, BCR-Abl tyrosine kinase, or KIT tyrosine kinase.

2. A pharmaceutical composition for treating or preventing drug-resistant leukemia with photodynamic therapy (PDT), comprising 5-aminolevulinic acid (5-ALA) as shown in the following formula (1): 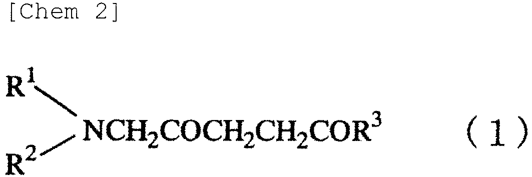 (wherein, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof.

3. The pharmaceutical composition according to claim 2, wherein the leukemia is adult T-cell leukemia.

4. The pharmaceutical composition of according to any one of claims 1 to 3, wherein the drug is lenalidomide or mogamulizumab.

5. The pharmaceutical composition according to any one of claims 1 to 3, wherein the drug is tretinoin.

6. The pharmaceutical composition according to any one of claims 1 to 3, wherein the drug is imatinib.

7. A pharmaceutical composition for treating or preventing a cancer with photodynamic therapy (PDT), comprising 5-aminolevulinic acid (5-ALA) as shown in the following formula (1): 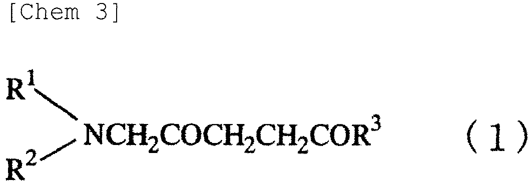 (wherein, each R¹ and R² are, identically or differently, hydrogen atom, alkyl group, acyl group, alkoxycarbonyl group, aryl group, or aralkyl group, and R³ is hydroxy group, alkoxy group, acyloxy group, alkoxycarbonyloxy group, aryloxy group, aralkyloxy group or amino group), or derivative thereof, or salt thereof, wherein the pharmaceutical composition is for use in combination with at least one of lenalidomide, mogamulizumab, tretinoin, and imatinib.

8. The pharmaceutical composition according to any one of claims 1 to 7, wherein the cancer is a hematological cancer.

9. The pharmaceutical composition according to any one of claims 1 to 8, wherein the PDT light irradiation is performed *in vitro.*
